# EUROPEAN PATENT SPECIFICATION

(11) **EP 1 653 964 B1**
(45) Date of publication and mention of the grant of the patent: **24.09.2008**
(21) Application number: 04743287.7
(22) Date of filing: 08.07.2004
(51) Int. Cl.: A61K 31/517, A61P 35/00

(54) **CANCER COMBINATION THERAPY COMPRISING AZD2171 AND ZD1839**
KOMBINATIONSTHERAPIE GEGEN KREBSERKRANKUNGEN MIT AZD2171 UND ZD1839
POLYTHERAPIE POUR LE CANCER CONTENANT AZD2171 ET ZD1839

(30) Priority: 10.07.2003 GB 0316127
(43) Date of publication of application: 10.05.2006
(73) Proprietor: AstraZeneca AB, 151 85 Södertälje (SE)
(72) Inventor: WEDGE, Stephen, Robert AstraZeneca R & D Alderley, Macclesfield Cheshire SK10 4TG (GB)
(86) International application number: PCT/GB2004/002944
(87) International publication number: WO 2005/004872

(56) References cited:
- WO-A-01/74360
- WO-A-03/064413

## Description

The present invention relates to a pharmaceutical composition comprising AZD2171 and ZD1839; to a kit comprising AZD2171 and ZD1839; and to the use of AZD2171 and ZD1839 in the manufacture of a medicament for use in the production of an antiangiogenic and/or vascular permeability reducing effect in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor-A (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36:139-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1, the kinase insert domain-containing receptor, KDR (also referred to as Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem., 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Application Publication No. WO 00/47212. In WO 00/47212 compounds are described which possess activity against VEGF receptor tyrosine kinase (VEGF RTK). Alternative quinazoline derivatives which are inhibitors of VEGF receptor tyrosine kinase are described in International Patent Application Publication No. WO 03/64413.

AZD2171 is 4-(4-fluoro-2-methylindol-5-yloxy)-6-methoxy-7-(3-(pyrrolidin-1-yl)propoxy)quinazoline:

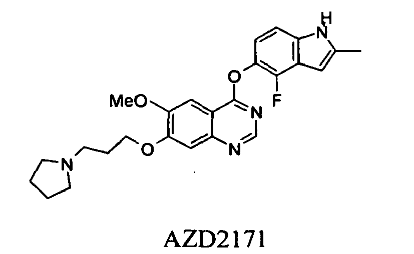

AZD2171 is Example 240 of WO 00/47212. ZD2171 is a very potent inhibitor of KDR and also has some activity against Flt-1. The IC₅₀ value for AZD2171 inhibition of KDR and Flt-1 tyrosine kinase activity, in recombinant enzyme assays *in vitro,* is < 2nM and 5nM respectively. AZD2171 has been shown to elicit broad-spectrum anti-tumour activity in a range of models following once-daily oral administration.

In WO 00/47212 it is stated that compounds of the invention:
"may be applied as a sole therapy or may involve, in addition to a compound of the invention, one or more other substances and/or treatments. Such conjoint treatment may be achieved by way of the simultaneous, sequential or separate administration of the individual components of the treatment."

WO 00/47212 then goes on to describe examples of such conjoint treatment including surgery, radiotherapy and various types of chemotherapeutic agent including inhibitors of growth factor function.

Nowhere in WO 00/47212 does it suggest the combination of a compound of the invention and an epidermal growth factor receptor tyrosine kinase inhibitor for the treatment of any disease state including cancer.

Nowhere in WO 00/47212 is the specific combination of AZD2171 and ZD1839 suggested.

Nowhere in WO 00/47212 does it state that use of any compound of the invention therein with other treatments will produce surprisingly beneficial effects.

An alternative combination of an AZD2171 with an anti-hypertensive agent is disclosed in International Patent Application Publication No. WO 01/74360.

Unexpectedly and surprisingly we have now found that the particular compound AZD2171 used in combination with a particular selection from the broad description of combination therapies listed in WO 00/47212, namely with ZD1839, produces significantly better effects than any one of AZD2171 and ZD1839 used alone. In particular, AZD2171 used in combination with ZD1839 produces significantly better effects on solid tumours than any one of AZD2171 and ZD1839 used alone.

ZD1839 is *N*-(3-chloro-4-fluorophenyl)-7-methoxy-6-(3-morpholinopropoxy)quinazolin-4-amine:

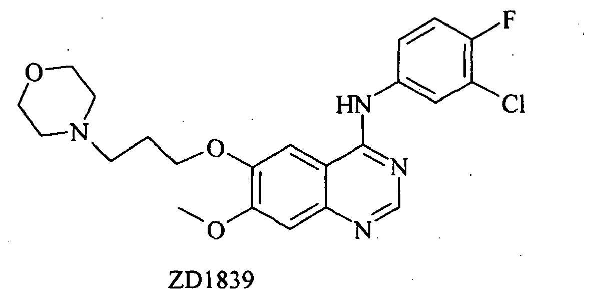

ZD1839 is also known as Iressa^{TM} (Trademark of AstraZeneca UK Limited) and it is an epidermal growth factor receptor (EGFR) tyrosine kinase inhibitor (TKI). ZD1839 is described in International Patent Application Publication No. WO 96/33980.

In recent years it has been discovered that certain growth factor tyrosine kinase enzymes are important in the transmission of biochemical signals which initiate cell replication. They are large proteins which span the cell membrane and possess an extracellular binding domain for growth factors, for example the epidermal growth factor receptor (EGFR) which binds epidermal growth factor (EGF), and an intracellular portion which functions as a kinase to phosphorylate tyrosine amino acids in proteins and hence to influence cell proliferation.

EGFR is a member of the erbB family of receptor tyrosine kinases, which includes EGFR, erbB2, erbB3 and erbB4, and it is known that these receptor tyrosine kinases are frequently involved in driving the proliferation and survival of tumour cells (reviewed in Olayioye et al., EMBO J., 2000, 19, 3159). One mechanism by which this can be accomplished is by overexpression of the receptor at the protein level, generally as a result of gene amplification. This has been observed in many common human cancers (reviewed in Klapper et al., Adv. Cancer Res., 2000, 77, 25), such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian cancer (Hellstrom et al., Cancer Res., 2001, 61, 2420), head and neck cancer (Shiga et al., Head Neck, 2000, 22, 599) and pancreatic cancer (Ovotny et al., Neoplasma, 2001, 48, 188).

It is widely believed that as a consequence of the dysfunctional regulation of one or more of these receptors many tumours become clinically more aggressive and this correlates with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as selective inhibitors of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, the use of inhibitory antibodies against EGFR and erbB2 (c-225 and trastuzumab respectively) has proven to be beneficial in the clinic for the treatment of selected solid tumours (reviewed in Mendelsohn et al, 2000, Oncogene, 19, 6550-6565).

It is believed that members of the erbB type receptor tyrosine kinase family may be implicated in a number of non-malignant proliferative disorders because amplification and/or activity of erbB receptor tyrosine kinases has been detected in psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders involving excessive cellular proliferation.

It is known from International Patent Application Publication No. WO 96/33980 that ZD1839 possesses EGF RTK inhibitory activity (J R Woodburn et al. in Proc. Amer. Assoc. Canc. Res., 1997, 38, 633 and Pharmacol. Ther., 1999, 82, 241-250) and is an inhibitor of the proliferation of cancer tissue.

It is stated in WO 96/33980 that compounds of the invention, which include ZD1839, may be given conjointly with other cancer therapies. It states therein "The anti-proliferative treatment defined hereinbefore may be applied as a sole therapy or may involve, in addition to the quinazoline derivative of the invention, one or more other anti-tumour substances, for example cytotoxic or cytostatic anti-tumour substances, for example those selected from, for example, mitotic inhibitors, for example vinblastine, vindesine and vinorelbine; tubulin disassembly inhibitors such as taxol; alkylating agents, for example cis-platin, carboplatin and cyclophosphamide; antimetabolites, for example 5-fluorouracil, tegafur, methotrexate, cytosine arabinoside and hydroxyurea, or, for example, one of the preferred antimetabolites disclosed in European Patent Application No. 239362 such as N- {5-[N-(3,4-dihydro-2-methyl-4-oxoquinazolin-6-ylmethyl)-N-methylamino]-2-thenoyl}-L-glutamic acid; intercalating antibiotics, for example adriamycin, mitomycin and bleomycin; enzymes, for example asparaginase; topoisomerase inhibitors, for example etoposide and camptothecin; biological response modifiers, for example interferon; anti-hormones, for example antioestrogens such as tamoxifen, for example antiandrogens such as 4'-cyano-3-(4-fluorophenylsulphonyl)-2-hydroxy-2-methyl-3'-(trifluoromethyl)-propionanilide or, for example LHRH antagonists or LHRH agonists such as goserelin, leuprorelin or buserelin and hormone synthesis inhibitors, for example aromatase inhibitors such as those disclosed in European Patent Application No. 0296749, for example 2,2'-[5-(1H-1,2,4- triazol-1-ylmethyl)-1,3-phenylene]bis(2-methylpropionitrile), and, for example, inhibitors of 5α-reductase such as 17β-(N-tert-butylcarbamoyl)-4-aza-5α-androst-1-en-3-one."

Nowhere in WO 96/33980 does it suggest any combination of an EGF RTK inhibitor with a VEGF RTK inhibitor for the treatment of any disease state including cancer.

International Patent Application Publication No. WO 02/41882 describes, generally, combinations of agents which decrease VEGF activity and agents which decrease EGF activity. A study has been carried out examining co-administration of antibodies to KDR and EGFR (Shaheen, R.M., et al., Brit. J. Cancer, 2001, 85, 584-589).

Unexpectedly and surprisingly we have now found that the particular compound AZD2171 used in combination with the particular compound ZD1839, produces significantly better anti-tumour effects than each of AZD2171 and ZD 1839 used alone.

Anti-cancer effects of a method of treatment of the present invention include, but are not limited to, anti-tumour effects, the response rate, the time to disease progression and the survival rate. Anti-tumour effects of a method of treatment of the present invention include but are not limited to, inhibition of tumour growth, tumour growth delay, regression of tumour, shrinkage of tumour, increased time to regrowth of tumour on cessation of treatment, slowing of disease progression. It is expected that when a method of treatment of the present invention is administered to a warm-blooded animal such as a human, in need of treatment for cancer, with or without a solid tumour, said method of treatment will produce an effect, as measured by, for example, one or more of: the extent of the anti-tumour effect, the response rate, the time to disease progression and the survival rate. Anti-cancer effects include prophylactic treatment as well as treatment of existing disease.

According to an aspect of the invention there is provided a pharmaceutical composition which comprises AZD2171 or a pharmaceutically acceptable salt thereof, and ZD1839 or a pharmaceutically acceptable salt thereof, in association with a pharmaceutically acceptable excipient or carrier.

According to a further aspect of the present invention there is provided a combination product comprising AZD2171 or a pharmaceutically acceptable salt thereof and ZD1839 or a pharmaceutically acceptable salt thereof, for use in a method of treatment of a human or animal body by therapy.

According to a further aspect of the present invention there is provided a kit comprising AZD2171 or a pharmaceutically acceptable salt thereof, and ZD1839 or a pharmaceutically acceptable salt thereof.

According to a further aspect of the present invention there is provided a kit comprising:
a) AZD2171 or a pharmaceutically acceptable salt thereof in a first unit dosage form;
b) ZD1839 or a pharmaceutically acceptable salt thereof in a second unit dosage form; and
c) container means for containing said first and second dosage forms.

According to a further aspect of the present invention there is provided a kit comprising:
a) AZD2171 or a pharmaceutically acceptable salt thereof, together with a pharmaceutically acceptable excipient or carrier, in a first unit dosage form;
b) ZD1839 or a pharmaceutically acceptable salt thereof together with a pharmaceutically acceptable excipient or carrier, in a second unit dosage form; and
c) container means for containing said first and second dosage forms.

According to a further aspect of the present invention there is provided the use of AZD2171 or a pharmaceutically acceptable salt thereof and ZD1839 or a pharmaceutically acceptable salt thereof in the manufacture of a medicament for use in the production of an antiangiogenic and/or vascular permeability reducing effect in a warm-blooded animal such as a human.

According to a further aspect of the present invention there is provided the use of AZD2171 or a pharmaceutically acceptable salt thereof and ZD 1839 or a pharmaceutically acceptable salt thereof in the manufacture of a medicament for use in the production of an anti-cancer effect in a warm-blooded animal such as a human.

According to a further aspect of the present invention there is provided the use of AZD2171 or a pharmaceutically acceptable salt thereof and ZD 1839 or a pharmaceutically acceptable salt thereof in the manufacture of a medicament for use in the production of an anti-tumour effect in a warm-blooded animal such as a human.

According to a further aspect of the present invention there is provided a therapeutic combination treatment comprising the administration of an effective amount of AZD2171 or a pharmaceutically acceptable salt thereof, optionally together with a pharmaceutically acceptable excipient or carrier, and the simultaneous, sequential or separate administration of an effective amount of ZD 183 9 or a pharmaceutically acceptable salt thereof; wherein ZD1839 may optionally be administered together with a pharmaceutically acceptable excipient or carrier, to a warm-blooded animal such as a human in need of such therapeutic treatment.

Such therapeutic treatment includes an antiangiogenic and/or vascular permeability effect, an anti-cancer effect and an anti-tumour effect.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent, in addition to a combination treatment of the invention.

Surgery may comprise the step of partial or complete tumour resection, prior to, during or after the administration of the combination treatment with AZD2171 described herein.

Other chemotherapeutic agents for optional use with a combination treatment of the present invention include those described in WO 00/47212 which is incorporated herein by reference. Such chemotherapy may cover five main categories of therapeutic agent:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.

The administration of a triple combination of AZD2171, ZD1839 and ionising radiation may produce effects, such as anti-tumour effects, greater than those achieved with any of AZD2171, ZD1839 and ionising radiation used alone, greater than those achieved with the combination of AZD2171 and ZD1839, greater than those achieved with the combination of AZD2171 and ionising radiation and greater than those achieved with the combination of ZD1839 and ionising radiation.

According to the present invention there is provided a method for the production of an antiangiogenic and/or vascular permeability reducing effect in a warm-blooded animal such as a human, which comprises administering to said animal an effective amount of AZD2171 or a pharmaceutically acceptable salt thereof, before, after or simultaneously with an effective amount of ZD1839 or a pharmaceutically acceptable salt thereof and before, after or simultaneously with an effective amount of ionising radiation.

According to a further aspect of the present invention there is provided a method for the treatment of a cancer in a warm-blooded animal such as a human, which comprises administering to said animal an effective amount of AZD2171 or a pharmaceutically acceptable salt thereof, before, after or simultaneously with an effective amount of ZD1839 or a pharmaceutically acceptable salt thereof and before, after or simultaneously with an effective amount of ionising radiation.

According to a further aspect of the present invention there is provided a method for the treatment of a cancer involving a solid tumour in a warm-blooded animal such as a human, which comprises administering to said animal an effective amount of AZD2171 or a pharmaceutically acceptable salt thereof, before, after or simultaneously with an effective amount of ZD1839 or a pharmaceutically acceptable salt thereof and before, after or simultaneously with an effective amount of ionising radiation.

According to a further aspect of the present invention there is provided a method for the production of an antiangiogenic and/or vascular permeability reducing effect in a warm-blooded animal such as a human, which comprises administering to said animal an effective amount of AZD2171 or a pharmaceutically acceptable salt thereof, before, after or simultaneously with an effective amount of ZD1839 or a pharmaceutically acceptable salt thereof and before, after or simultaneously with an effective amount of ionising radiation, wherein AZD2171 and ZD1839 may each optionally be administered together with a pharmaceutically acceptable excipient or carrier.

According to a further aspect of the present invention there is provided a method for the treatment of a cancer in a warm-blooded animal such as a human, which comprises administering to said animal an effective amount of AZD2171 or a pharmaceutically acceptable salt thereof, before, after or simultaneously with an effective amount of ZD1839 or a pharmaceutically acceptable salt thereof and before, after or simultaneously with an effective amount of ionising radiation, wherein AZD2171 and ZD1839 may each optionally be administered together with a pharmaceutically acceptable excipient or carrier.

According to a further aspect of the present invention there is provided a method for the treatment of a cancer involving a solid tumour in a warm-blooded animal such as a human, which comprises administering to said animal an effective amount of AZD2171 or a pharmaceutically acceptable salt thereof, before, after or simultaneously with an effective amount of ZD 1839 or a pharmaceutically acceptable salt thereof and before, after or simultaneously with an effective amount of ionising radiation, wherein AZD2171 and ZD1839 may each optionally be administered together with a pharmaceutically acceptable excipient or carrier.

According to a further aspect of the present invention there is provided the use of AZD2171 or a pharmaceutically acceptable salt thereof and ZD1839 or a pharmaceutically acceptable salt thereof in the manufacture of a medicament for use in the production of an antiangiogenic and/or vascular permeability reducing effect in a warm-blooded animal such as a human which is being treated with ionising radiation.

According to a further aspect of the present invention there is provided the use of AZD2171 or a pharmaceutically acceptable salt thereof and ZD 1839 or a pharmaceutically acceptable salt thereof in the manufacture of a medicament for use in the production of an anti-cancer effect in a warm-blooded animal such as a human which is being treated with ionising radiation.

According to a further aspect of the present invention there is provided the use of AZD2171 or a pharmaceutically acceptable salt thereof and ZD1839 or a pharmaceutically acceptable salt thereof in the manufacture of a medicament for use in the production of an anti-tumour effect in a warm-blooded animal such as a human which is being treated with ionising radiation.

According to a further aspect of the present invention there is provided a therapeutic combination treatment comprising the administration of an effective amount of AZD2171 or a pharmaceutically acceptable salt thereof, optionally together with a pharmaceutically acceptable excipient or carrier, and the administration of an effective amount of ZD1839 or a pharmaceutically acceptable salt thereof, optionally together with a pharmaceutically acceptable excipient or carrier and the administration of an effective amount of ionising radiation, to a warm-blooded animal such as a human in need of such therapeutic treatment wherein the AZD2171, ZD 1839 and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising AZD2171 and ZD1839. For example said ionising radiation may be given to said warm-blooded animal such as a human within the period of a week before to a week after the administration of a medicament or combination treatment comprising AZD2171 and ZD1839. This means that AZD2171, ZD1839 and ionising radiation may be administered separately or sequentially in any order, or may be administered simultaneously. The warm-blooded animal may experience the effect of each of AZD2171, ZD1839 and radiation simultaneously.

According to one aspect of the present invention the ionising radiation is administered before one of AZD2171 and ZD1839 or after one of AZD2171 and ZD1839.

According to one aspect of the present invention the ionising radiation is administered before both AZD2171 and ZD1839 or after both AZD2171 and ZD1839.

According to one aspect of the present invention AZD2171 is administered to a warm-blooded animal after the animal has been treated with ionising radiation.

In another aspect of the present invention AZD2171 and ZD1839 are dosed daily continuously for a longer period of time during which time ionising radiation is administered periodically, that is for a few days, for example 1, 2, 3, 4 or 5 days at a time.

According to another aspect of the present invention the effect of a method of treatment of the present invention is expected to be at least equivalent to the addition of the effects of each of the components of said treatment used alone, that is, of each of AZD2171 and ZD1839 or of each of AZD2171, ZD1839 and ionising radiation used alone.

According to another aspect of the present invention the effect of a method of treatment of the present invention is expected to be greater than the addition of the effects of each of the components of said treatment used alone, that is, of each of AZD2171 and ZD1839 or of each of AZD2171, ZD1839 and ionising radiation, used alone.

According to another aspect of the present invention the effect of a method of treatment of the present invention is expected to be a synergistic effect.

It should also be appreciated that according to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with AZD2171 or ZD1839 or ionising radiation alone. Further, the effect of the combination treatment is synergistic if a beneficial effect is obtained in a group of patients that does not respond (or responds poorly) to AZD2171 or ZD1839 or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of AZD2171 or ZD1839 or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments of the present invention as defined herein are of interest for their antiangiogenic and/or vascular permeability effects. Combination treatments of the invention are expected to be useful in the prophylaxis and treatment of a wide range of disease states where inappropriate angiogenesis occurs including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, lymphoedema, endometriosis, dysfunctional uterine bleeding and ocular diseases with retinal vessel proliferation including macular degeneration. In particular such combination treatments of the invention are expected to slow advantageously the growth of primary and recurrent solid tumours of, for example, the colon, breast, prostate, lungs and skin. More especially combination treatments of the present invention are expected to slow advantageously the growth of tumours in lung cancer, particularly non-small cell lung cancer (NSCLC). More particularly such combination treatments of the invention are expected to inhibit any form of cancer associated with VEGF including leukaemia, mulitple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), kidney, breast, prostate, lung, vulva and skin, particularly NSCLC.

In another aspect of the present invention AZD2171 and ZD1839, optionally with ionising radiation, are expected to inhibit the growth of those primary and recurrent solid tumours which are associated with EGF especially those tumours which are significantly dependent on EGF for their growth and spread.

In another aspect of the present invention AZD2171 and ZD1839, optionally with ionising radiation, are expected to inhibit the growth of those primary and recurrent solid tumours which are associated with both VEGF and EGF especially those tumours which are significantly dependent on VEGF and EGF for their growth and spread.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the AZD2171 and ZD1839 of the combination treatment may be delivered endoscopically, intratracheally, intralesionally, percutaneously, intravenously, subcutaneously, intraperitoneally or intratumourally. Preferably AZD2171 is administered orally. Preferably ZD1839 is administered orally. In general the compositions described herein may be prepared in a conventional manner using conventional excipients. The compositions of the present invention are advantageously presented in unit dosage form.

AZD2171 will normally be administered to a warm-blooded animal at a unit dose within the range 1-50mg per square metre body area of the animal, for example approximately 0.03-1.5 mg/kg in a human. A unit dose in the range, for example, 0.01-1.5mg/kg, preferably, preferably 0.03-0.5mg/kg is envisaged and this is normally a therapeutically-effective dose. A unit dosage form such as a tablet or capsule will usually contain, for example 1-50mg of active ingredient. Preferably a daily dose in the range of 0.03-0.5mg/kg is employed.

For ZD 1839, a conventional tablet formulation may be used for oral administration to humans containing 50 mg, 100 mg, 250 mg or 500 mg of active ingredient. Conveniently the daily oral dose of ZD 1839 is, for example, in the range 25 to 750 mg, preferably in the range 50 to 600 mg, more preferably in the range 100 to 400mg.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

The present invention relates to combinations of ZD1839 or a salt thereof with AZD2171 or with a salt of AZD2171.

Salts of ZD 1839 for use in pharmaceutical compositions will be pharmaceutically acceptable salts, but other salts may be useful in the production of ZD 1839 and its pharmaceutically acceptable salts. Salts include, for example, an acid-addition salt of ZD1839, for example, a mono- or di-acid-addition salt with, for example, an inorganic or organic acid, for example hydrochloric, hydrobromic, sulphuric, phosphoric, trifluoroacetic, citric, maleic, tartaric, fumaric, methanesulphonic or 4-toluenesulphonic acid.

Salts of AZD2171 for use in pharmaceutical compositions will be pharmaceutically acceptable salts, but other salts maybe useful in the production of AZD2171 and its pharmaceutically acceptable salts. Pharmaceutically acceptable salts may, for example, include acid addition salts. Such acid addition salts include for example salts with inorganic or organic acids affording pharmaceutically acceptable anions such as with hydrogen halides or with sulphuric or phosphoric acid, or with trifluoroacetic, citric or maleic acid. In addition pharmaceutically acceptable salts may be formed with an inorganic or organic base which affords a pharmaceutically acceptable cation. Such salts with inorganic or organic bases include for example an alkali metal salt, such as a sodium or potassium salt and an alkaline earth metal salt such as a calcium or magnesium salt.

AZD2171 may be synthesised according to the processes described in WO 00/47212, in particular those described in Example 240 of WO 00/47212.

ZD 1839 may be synthesised according to any of the known processes for making ZD1839. For example ZD1839 may be made according to the processes described in WO 96/33980 (See Examples 1,10 and 24-31).

The following tests may be used to demonstrate the activity of AZD2171 in combination with ZD1839.

### Human A431 vulval carcinoma tumour xenografts in Nude mice

5 x 10⁶ A431 tumour cells in 0.1 ml of serum free Dulbecco's Modified Eagle's Medium (DMEM) were injected subcutaneously (s.c.) into the flank of each athymic (*nu*/*nu* genotype) mouse. Eleven days after inoculation tumours were excised and dissected to generate cubic tumour fragments of 0.5 - 1 mm³, which were implanted into further nude mice for a therapy experiment. Tumour volumes were assessed by bilateral Vernier caliper measurement and, taking length to be the longest diameter across the tumour and width the corresponding perpendicular, calculated using the formula (length x width) x the square root of (length x width) x (π/6). Twenty eight days after implantation when tumours reached a mean volume of 0.9cm³, mice were treated with AZD2171 (3 mg/kg/administration), ZD1839 (50 mg/kg/administration), or a combination thereof, orally (p.o.) daily for 24 days (day 28 - 52). AZD2171, ZD1839 or the combination, were dosed at 0.1ml/10g body weight, as suspensions in 1% polysorbate 80 (i.e. a 1% (v/v) solution of polyoxyethylene (20) sorbitan mono-oleate in deionised water). Inhibition of tumour growth from the start of treatment was assessed on day 45 (the point at which control animals were removed from the experiment because of tumour burden) by comparison of the differences in tumor volume between control and treated groups. In addition, the number of tumour regressions following 24 days of treatment was ascertained (tumour regression being evident if the tumour volume at day 52 was smaller than the pre-treatment value on day 28).

**Table I**

| Treatment | Mean % Inhibition of tumour growth following 17 days of treatment (treatment from day 28 to day 45) [P value by two-tailed t-test] | Number of tumours regressing after 24 days of treatment (treatment from day 28 to day 52) |
|---|---|---|
| AZD2171 (3 mg/kg/day, days 28 - 53, p.o.) | 95 % (P<0.001) | 2/10 |
| ZD1839 (50 mg/kg/day, days 28 - 53, p.o.) | 94% (P<0.001) | 2/10 |
| AZD2171 + ZD1839 | 141% (P<0.001) | 10/10 |
| | (i.e. 41% tumour regression) | |

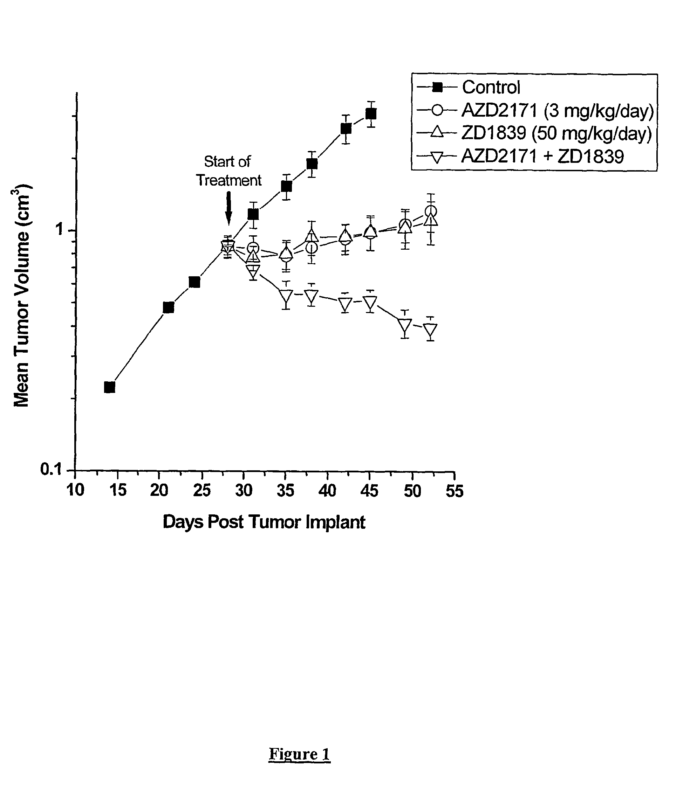

The combination of AZD2171 with ZD1839 produced a greater inhibition of tumour growth than each of AZD2171 and ZD1839 alone (P<0.005 versus either AZD2171 or ZD1839 alone at day 52; one-tailed t-test). In contrast to treatment with AZD2171 or ZD1839 alone, regression was induced in all A431 vulval carcinoma xenografts treated with the combination of AZD2171 and ZD1839. The magnitude of tumour regression induced by the combination (calculated by comparing the pre-treatment tumour volume (day 28) with the volume following 24 days of treatment (day 52)), reached 55 ± 3 % (mean ± SE) by the end of the experiment.
The data is shown graphically in Figure 1.

### MMTV-neu transgenic model

The effect of combining AZD2171 with ZD1839 was also examined in MMTV-neu mice. These mice transgenically express an activated form of the rat neu (ErbB2) oncogene under the control of the mouse mammary tumor virus (MMTV) promoter. Following pregnancy, which induces activation of the promoter, MMTV-neu mice spontaneously develop multiple tumours within each mammary gland. Mice were selected for dosing when their largest mammary tumour reached a volume of ≥ 0.4 cm³. Mice were then chronically treated (orally, once-daily) with AZD2171 (1 or 3 mg/kg/day), ZD1839 (50 mg/kg/day) or combinations of both. Control animals were dosed with vehicle (1% polysorbate 80). Due to variability in the growth of spontaneous tumours, dosing commenced at different times relative to the assessment of tumour volume, which was determined twice weekly on all animals using calipers. To simplify the analysis, data within each group were collated in 3 day intervals (e.g. tumour volumes from mice receiving either 2, 3 or 4 doses of compound were pooled and the mean volume plotted against "day 3"). Data is plotted for measurements grouped over 3 day periods ie 2-4, 5-7, 8-10 etc. Statistical analysis was performed on measurements between 20 and 22 days of dosing. Previously measurable tumours which became unpalpable were assigned a value of 0.

Treatment with AZD2171 alone (1 or 3 mg/kg/day) inhibited the growth of established (≥0.4 cm³) MMTV-neu tumours significantly (P<0.05). Treatment with ZD1839 alone did not inhibit MMTV-neu tumour growth at a dose of 50 mg/kg/day, but has been found to elicit a significant anti-tumour effect when dosed at 100 mg/kg/day. Nonetheless, the combination of AZD2171 (1 or 3 mg/kg/day) with 50 mg/kg/day of ZD1839, produced a greater inhibition of tumour growth than either the respective dose of AZD2171 alone (1 or 3 mg/kg/day) or ZD1839 (50 mg/kg/day) alone (P≤ 0.002, one-tailed t test).

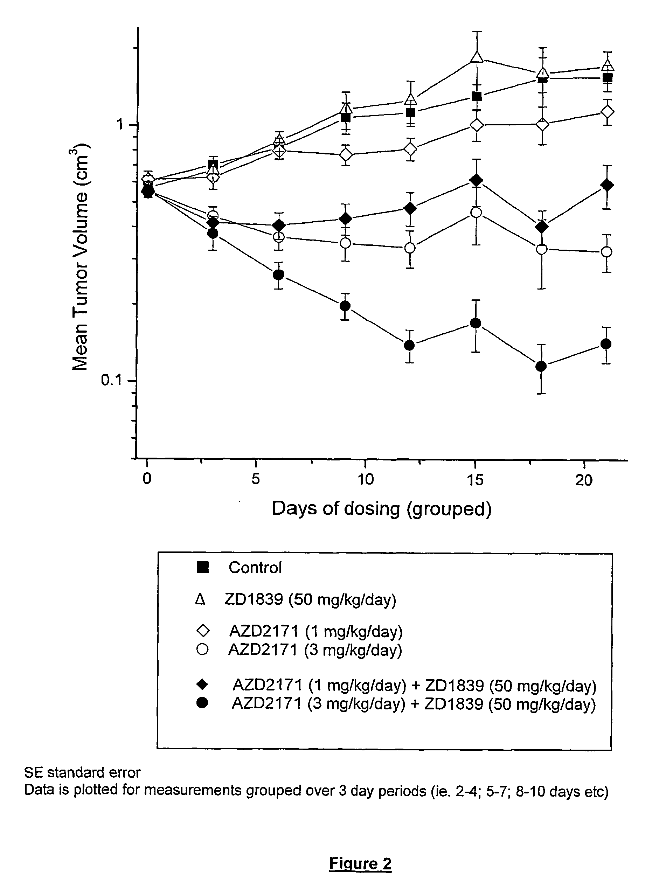

The data is shown graphically in Figure 2.

## Claims

1. A pharmaceutical composition which comprises AZD2171 or a pharmaceutically acceptable salt thereof, and ZD1839 or a pharmaceutically acceptable salt thereof, in association with a pharmaceutically acceptable excipient or carrier.

2. A kit comprising AZD2171 or a pharmaceutically acceptable salt thereof, and ZD1839 or a pharmaceutically acceptable salt thereof.

3. Use of AZD2171 or a pharmaceutically acceptable salt thereof and ZD 1839 or a pharmaceutically acceptable salt thereof in the manufacture of a medicament for use in the production of an antiangiogenic and/or vascular permeability reducing effect in a warm-blooded animal such as a human.

4. Use of AZD2171 or a pharmaceutically acceptable salt thereof and ZD1839 or a pharmaceutically acceptable salt thereof in the manufacture of a medicament for use in the production of an antiangiogenic and/or vascular permeability reducing effect in a warm-blooded animal such as a human which is being treated with ionising radiation.

5. Use of AZD2171 or a pharmaceutically acceptable salt thereof and ZD 1839 or a pharmaceutically acceptable salt thereof in the manufacture of a medicament for use in the production of an anti-cancer effect in a warm-blooded animal such as a human.

6. Use of AZD2171 or a pharmaceutically acceptable salt thereof and ZD1839 or a pharmaceutically acceptable salt thereof in the manufacture of a medicament for use in the production of an anti-cancer effect in a warm-blooded animal such as a human which is being treated with ionising radiation.

7. Use of AZD2171 or a pharmaceutically acceptable salt thereof and ZD1839 or a pharmaceutically acceptable salt thereof in the manufacture of a medicament for use in the production of an anti-tumour effect in a warm-blooded animal such as a human.

8. Use of AZD2171 or a pharmaceutically acceptable salt thereof and ZD1839 or a pharmaceutically acceptable salt thereof in the manufacture of a medicament for use in the production of an anti-tumour effect in a warm-blooded animal such as a human which is being treated with ionising radiation.

9. Use according to Claim 7 or Claim 8 wherein the anti-tumour effect is in a primary or recurrent solid tumour.

10. Use according to Claim 9 wherein the primary or recurrent solid tumour is selected from colon, breast, prostate, lung and skin.

11. Use according to Claim 7 or Claim 8 wherein the anti-tumour effect is in lung cancer.

12. Use according to Claim 11 wherein the lung cancer is non-small cell lung cancer.

## Patentansprüche

1. Pharmazeutische Zusammensetzung, die AZD2171 oder ein pharmazeutisch annehmbares Salz davon und ZD1839 oder ein pharmazeutisch annehmbares Salz davon zusammen mit einem pharmazeutisch annehmbaren Exzipienten oder Träger enthält.

2. Kit, enthaltend AZD2171 oder ein pharmazeutisch annehmbares Salz davon und Zu1839 oder ein pharmazeutisch annehmbares Salz davon.

3. Verwendung von AZD2171 oder einem pharmazeutisch annehmbaren Salz davon und ZD1839 oder einem pharmazeutisch annehmbaren Salz davon bei der Herstellung eines Medikaments zur Verwendung beim Hervorrufen eines antiangiogenen und/oder die vaskuläre Permeabilität vermindernden Effekts bei einem Warmblüter wie einem Menschen.

4. Verwendung von AZD2171 oder einem pharmazeutisch annehmbaren Salz davon und ZD1839 oder einem pharmazeutisch annehmbaren Salz davon bei der Herstellung eines Medikaments zur Verwendung beim Hervorrufen eines antiangiogenen und/oder die vaskuläre Permeabilität vermindernden Effekts bei einem Warmblüter wie einem Menschen, der mit ionisierender Strahlung behandelt wird.

5. Verwendung von AZD2171 oder einem pharmazeutisch annehmbaren Salz davon und ZD1839 oder einem pharmazeutisch annehmbaren Salz davon bei der Herstellung eines Medikaments zur Verwendung beim Hervorrufen einer Antikrebswirkung bei einem Warmblüter wie einem Menschen.

6. Verwendung von AZD2171 oder einem pharmazeutisch annehmbaren Salz davon und ZD1839 oder einem pharmazeutisch annehmbaren Salz davon bei der Herstellung eines Medikaments zur Verwendung beim Hervorrufen einer Antikrebswirkung bei einem Warmblüter wie einem Menschen, der mit ionisierender Strahlung behandelt wird.

7. Verwendung von AZD2171 oder einem pharmazeutisch annehmbaren Salz davon und ZD1839 oder einem pharmazeutisch annehmbaren Salz davon bei der Herstellung eines Medikaments zur Verwendung beim Hervorrufen einer Antitumorwirkung bei einem Warmblüter wie einem Menschen.

8. Verwendung von AZD2171 oder einem pharmazeutisch annehmbaren Salz davon und ZD1839 oder einem pharmazeutisch annehmbaren Salz davon bei der Herstellung eines Medikaments zur Verwendung beim Hervorrufen einer Antitumorwirkung bei einem Warmblüter wie einem Menschen, der mit ionisierender Strahlung behandelt wird.

9. Verwendung nach Anspruch 7 oder 8, wobei die Antitumorwirkung bei einem primären oder rezidiven festen Tumor auftritt.

10. Verwendung nach Anspruch 9, wobei der primäre bzw. rezidive feste Tumor ausgewählt ist aus Kolon, Brust, Prostata, Lunge und Haut.

11. Verwendung nach Anspruch 7 oder 8, wobei die Antitumorwirkung bei Lungenkrebs auftritt.

12. Verwendung nach Anspruch 11, wobei es sich bei dem Lungenkrebs um nichtkleinzelligen Lungenkrebs handelt.

## Revendications

1. Composition pharmaceutique comprenant AZD2171 ou un sel pharmaceutiquement acceptable de celui-ci, et ZD1839 ou un sel pharmaceutiquement acceptable de celui-ci, en association avec un excipient ou un support pharmaceutiquement acceptable.

2. Kit comprenant AZD2171 ou un sel pharmaceutiquement acceptable de celui-ci, et ZD1839 ou un sel pharmaceutiquement acceptable de celui-ci.

3. Utilisation de AZD2171 ou d'un sel pharmaceutiquement acceptable de celui-ci et de ZD1839 ou d'un sel pharmaceutiquement acceptable de celui-ci, dans la fabrication d'un médicament destiné à être utilisé dans la production d'un effet anti-angiogénique et/ou réducteur de la perméabilité vasculaire chez un animal à sang chaud tel que l'homme.

4. Utilisation de AZD2171 ou d'un sel pharmaceutiquement acceptable de celui-ci et de ZD1839 ou d'un sel pharmaceutiquement acceptable de celui-ci, dans la fabrication d'un médicament destiné à être utilisé dans la production d'un effet anti-angiogénique et/ou réducteur de la perméabilité vasculaire chez un animal à sang chaud tel que l'homme traité par des radiations ionisantes.

5. Utilisation de AZD2171 ou d'un sel pharmaceutiquement acceptable de celui-ci et de ZD1839 ou d'un sel pharmaceutiquement acceptable de celui-ci, dans la fabrication d'un médicament destiné à être utilisé dans la production d'un effet anti-cancéreux chez un animal à sang chaud tel que l'homme.

6. Utilisation de AZD2171 ou d'un sel pharmaceutiquement acceptable de celui-ci et de ZD1839 ou d'un sel pharmaceutiquement acceptable de celui-ci, dans la fabrication d'un médicament destiné à être utilisé dans la production d'un effet anti-cancéreux chez un animal à sang chaud tel que l'homme traité par des radiations ionisantes.

7. Utilisation de AZD2171 ou d'un sel pharmaceutiquement acceptable de celui-ci et de ZD1839 ou d'un sel pharmaceutiquement acceptable de celui-ci dans la fabrication d'un médicament destiné à être utilisé dans la production d'un effet anti-tumoral chez un animal à sang chaud tel que l'homme.

8. Utilisation de AZD2171 ou d'un sel pharmaceutiquement acceptable de celui-ci et de ZD1839 ou d'un sel pharmaceutiquement acceptable de celui-ci dans la fabrication d'un médicament destiné à être utilisé dans la production d'un effet anti-tumoral chez un animal à sang chaud tel que l'homme traité par des radiations ionisantes.

9. Utilisation selon la revendication 7 ou la revendication 8, **caractérisée en ce que** l'effet anti-tumoral est dans une tumeur solide primaire ou récidivante.

10. Utilisation selon la revendication 9, **caractérisée en ce que** la tumeur solide primaire ou récidivante est choisie parmi le colon, le sein, la prostate, le poumon et la peau.

11. Utilisation selon la revendication 7 ou la revendication 8, **caractérisée en ce que** l'effet anti-tumoral est lors du cancer du poumon.

12. Utilisation selon la revendication 11, **caractérisée en ce que** le cancer du poumon est le cancer du poumon non à petites cellules.
